(19) Europäisches Patentamt
European Patent Office
Office européen des brevets

(11) Veröffentlichungsnummer: **0 239 931 B1**

(12) # EUROPÄISCHE PATENTSCHRIFT

(45) Veröffentlichungstag der Patentschrift: **18.03.92**

(51) Int. Cl.5: **C07D 257/04**, C12Q 1/26

(21) Anmeldenummer: **87104466.5**

(22) Anmeldetag: **26.03.87**

(54) **Verfahren und Reagenz zur Bestimmung von Substraten oder Enzymaktivitäten.**

(30) Priorität: **04.04.86 DE 3611227**

(43) Veröffentlichungstag der Anmeldung:
**07.10.87 Patentblatt 87/41**

(45) Bekanntmachung des Hinweises auf die Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 037 466**
**EP-A- 0 127 179**
**EP-A- 0 167 973**
**DE-A- 2 147 466**
**GB-A- 738 585**
**GB-A- 1 513 488**
**US-A- 2 713 581**
**US-A- 3 071 465**

**Clin. Chem. 27, 1686 (1981)**

(73) Patentinhaber: **BOEHRINGER MANNHEIM GMBH**
**Sandhofer Strasse 116**
**W-6800 Mannheim 31(DE)**

(72) Erfinder: **Town, Michael-Harold, Dr.rer.nat.**
**Waldstrasse 45**
**W-8125 Oberhausen(DE)**
Erfinder: **Siedel, Joachim, Dr.rer.nat.**
**Bahnhofstrasse 6**
**W-8131 Bernried(DE)**
Erfinder: **Ziegenhorn, Joachim Dr.rer.nat.**
**Ina-Seidel-Weg 1**
**W-8130 Starnberg(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur quantitativen Bestimmung von Substraten oder Enzymaktivitäten unter Anwendung einer Redox-Reaktion als Meßreaktion.

In der klinischen und pharmazeutischen Chemie, der Biochemie und Lebensmittelchemie werden zur Bestimmung von Substraten oder Enzymaktivitäten eine Vielzahl von Indikatormethoden verwendet. Von besonderer Bedeutung ist jedoch zum Beispiel die Änderung der Extinktion eines Redox-Farbindikatorsystems oder Änderungen im elektrischen Potential oder Strom, welche durch eine geeignete Elektrode gemessen werden können. Solche Redox-Reaktionen finden insbesondere breite Anwendung in der klinischen Chemie.

Sind in dem zur photometrischen Bestimmung eines bestimmten Substrats oder Enzyms eingesetzten Testsystem, welches auf der Anwendung einer Redox-Reaktion als Meßreaktion beruht, neben den interessierenden Redox-Partnern nun noch weitere reduzierende Substanzen vorhanden, so ist mit Störungen der Meßreaktion zu rechnen. Mit solchen Störungen ist bekanntermaßen, insbesondere bei Verwendung von biologischem Probenmaterial, wie Harn, Plasma oder Serum, zu rechnen, die reduzierende Substanzen sowohl endogenen als auch exogenen Ursprungs in signifikanten Mengen enthalten können. Bei endogenen Störsubstanzen ist hier vor allem Bilirubin zu nennen. Störende reduzierende Substanzen exogenen Ursprungs können zum Beispiel Ascorbat, verschiedene Medikamente oder deren Metaboliten sein. In biologischen Proben oft vorkommende und aufgrund ihrer reduzierenden Eigenschaften störende Medikamente sind zum Beispiel $\alpha$-Methyldopa oder Calciumdobesylat. Als störender Metabolit ist zum Beispiel Homogentisinsäure bekannt.

Störungen durch reduzierende Substanzen wirken sich besonders deutlich aus, wenn die Konzentration oder Aktivität des zu bestimmenden Substrats oder des Enzyms in der Probe relativ niederig ist und zum Erzielen einer ausreichenden Meßpräzision ein relativ hohes Probe-/Reagenz-Volumenverhältnis erforderlich ist. Dies ist beispielsweise bei der Bestimmung von Harnsäure, Creatinin oder Oxalat in Serum oder Plasma der Fall. Hier wird ein zu bestimmendes Substrat mit einem Enzym unter Wasserstoffperoxidbildung umgesetzt, wobei das entstehende Wasserstoffperoxid in Gegenwart von Peroxidase bei einer stöchiometrischen Farbstoffbildungsreaktion, wie z. B. der oxidativen Kupplung zweier Kupplungskomponenten, verbraucht wird.

Bisher war es nicht möglich, Störungen durch reduzierende Substanzen gleichermaßen sowohl rasch und effektiv als auch ohne zusätzliche Beeinträchtigung der zur Messung notwendigen Redox-Reaktion zu beseitigen. Hierzu sind in der Chemie stark oxidierende Substanzen üblich. Diese sind jedoch für enzymatische Bestimmungsmethoden in der klinischen und pharmazeutischen Chemie, der Biochemie oder der Lebensmittelchemie unbrauchbar, da sie auch Substrate und Enzyme angreifen und zerstören. Außerdem führen sie häufig zu Nebenreaktionen, die die Reagenzleerwert-Extinktion erhöhen.

Viele Metallsalze und deren Komplexe reagieren ebenfalls mit reduzierenden Substanzen unter Bildung der entsprechenden zwei- oder dreiwertigen Ionen. Diese hemmen häufig die Enzyme, die zur Indikatorreaktion benutzt werden. Darüberhinaus können die entstandenen reduzierten Metallionen in gleicher Weise stören, wie die sich in dem Probenmaterial befindlichen Störsubstanzen. Ein zusätzlicher Nachteil der Metallionen besteht darin, daß deren Oxidation unter stark sauren Bedingungen optimal ist. Unter diesen Bedingungen werden Enzyme meist zerstört.

Außer diesen herkömmlichen Verfahren kommt speziell für Ascorbinsäure der rasche oxidative Abbau von Ascorbat mit Luftsauerstoff in Frage. Dieser ist jedoch nur möglich unter stark alkalischen Bedingungen, unter denen Enzyme denaturiert oder inaktiviert werden.

Zur Vermeidung von Störungen von Redoxreaktionen durch Ascorbinsäure ist in EP-A-0 037 056 auch der Einsatz von Jodat beschrieben.

Als andere Möglichkeit wurde der Einsatz von Ascorbatoxidase als Katalysator für diese Reaktion vorgeschlagen (DE-OS 26 25 834). Dieses Verfahren kann aufgrund der Spezifität des Enzyms Ascorbat-Oxidase außer für Ascorbat nicht für andere reduzierende Störsubstanzen verwendet werden. Das pH-Optimum des Enzyms liegt bei pH 5 - 6 und ist damit weit von den pH-Optima vieler für klinische Tests wichtigen Enzyme entfernt. So erfolgt z. B. die Bestimmung von Creatinin oder Harnsäure bei pH 8. Bei diesem pH-Wert ist die Aktivität der Ascorbat-Oxidase nicht ausreichend für eine schnelle Entstörung. Außerdem wird dieses Enzym durch einige übliche Testkomponenten, wie Azid oder Chelatoren, wie zum Beispiel EDTA, gehemmt.

Es bestand daher Bedarf für ein universell anwendbares Verfahren und Reagenz zur Bestimmung von Substraten oder Enzymaktivitäten, welches Störungen durch reduzierende Substanzen in Redox-Reaktionen beseitigt, ohne jedoch die Meßreaktion nachteilig zu beeinflussen. Das Reagenz sollte darüber hinaus einfach in der Handhabung und auch preiswert sein. Aufgabe war es, diesen Bedarf zu befriedigen.

Die Aufgabe wird gelöst durch das erfindungsgemäße Verfahren zur Bestimmung von Substraten oder Enzymaktivitäten, bei dem dann, wenn eine Redox-Reaktion als Meßreaktion dient, in Gegenwart eines oder mehrerer zusätzlich zugegebener Tetrazoliumsalze gearbeitet wird, wobei die Tetrazoliumsalze und deren Formazane nicht oder nur in vernachlässigbarem Maß bei der Wellenlänge der Meßreaktion Licht absorbieren. Der Vorteil dieses Verfahrens besteht in der universellen, irreversiblen, leichten und schnellen Beseitigung reduzierender Substanzen, die unter den in den betreffenden Substrat- oder Enzymtests vorherrschenden Bedingungen stören. Dadurch wird eine störungsfreie Messung ermöglicht.

Tetrazoliumsalze und ihre Reduktion zu Formazanen sind schon seit Ende des letzten Jahrhunderts bekannt. Sie werden vorwiegend zum Nachweis von reduzierenden Substanzen in Chemie, Biochemie und Histochemie verwendet (vgl. z. B.: Bergmeyer, Methods of Enzymatic Analysis, Band I, Seite 199 ff [1984] oder Altmann, F. P., Progress in Histochemistry, 9, Seite 1 ff [1976] oder DE-A-21 47 466, GB-A-15 13 488, Clin. Chem. 27 1686 [1981]). Bisher wurde jedoch noch niemals in Betracht gezogen, eine als Farbindikator wirkende Redox-Reaktion mit einer Tetrazoliumsalz-Reduktion zu kombinieren, mit dem Ziel, diese Reduktion eines Tetrazoliumsalzes zu einem Formazan nicht als Nachweisreaktion, sondern als entstörende Reaktion auszunutzen. Das bisherige Interesse bestand darin, Tetrazoliumsalze zu finden, die bei Reduktion möglichst farbige Formazane bilden und deshalb als Farbindikatoren in Redoxreaktionen einzusetzen sind.

Um bei einer als Meßreaktion verwendeten, photometrisch verfolgbaren Redoxreaktion das Farbindikatorsystem nicht zu stören, müssen die zur Beseitigung störender reduzierender Substanzen verwendeten Tetrazoliumsalze solche Formazane bilden, die Licht nicht oder nur in vernachlässigbarem Ausmaß bei der Meßwellenlänge des eigentlichen Farbindikatorsystems absorbieren. Für die vor allem in der klinischen Chemie üblichen Farbindikatorsysteme mit maximalen Absorptionswellenlängen zwischen 500 und 600 nm kann deshalb erfindungsgemäß prinzipiell jedes Tetrazoliumsalz verwendet werden, welches in diesem Wellenlängenbereich nicht oder nur vernachlässigbar absorbiert.

Im Sinne der vorliegenden Erfindung haben sich vor allem Tetrazoliumsalze der allgemeinen Formel I

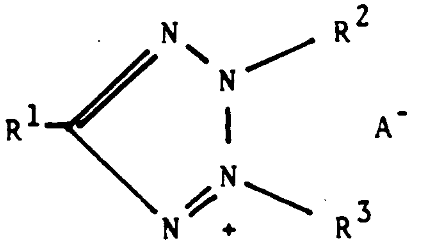

(I)

in der

- $R^1$ Wasserstoff, einen Carboxyl-, Alkyl-, Phenyl-, Nitrophenyl-, Dinitrophenyl-, carboxylsubstituierten Phenyl- oder einen Trialkylammoniumphenylrest,
- $R^2$ einen Phenyl-, Nitrophenyl-, Biphenylyl- oder Naphthylrest,
- $R^3$ einen Phenyl-, carboxylsubstituierten Phenyl-, einen carboxylsubstituierten Hydroxyphenyl- oder einen Dimethylthiazolylrest und
- $A^-$ ein übliches Gegenion

bedeuten, bewährt.

Ein Alkylrest in der Definition von $R^1$ besteht aus 1-10, bevorzugt 1-7 Kohlenstoffatomen. Besonders bevorzugt sind der Methyl-, Ethyl- und n-Butylrest.

Besonders bevorzugt als Nitrophenylrest in der Definition von $R^1$ und $R^2$ ist der para-Nitrophenylrest.

Unter einem in der Definition von $R^1$ genannten Dinitrophenylrest ist vor allem der 2,4-Dinitrophenylrest zu verstehen.

Carboxylsubstituierte Phenylreste in der Definition von $R^1$ und $R^3$ sind dann bevorzugt, wenn der Phenylrest in 4-Stellung durch eine Carboxylgruppe substituiert ist. In der Definition von $R^3$ sind darüberhinaus solche Reste dann ganz besonders bevorzugt, wenn der Phenylrest den Carboxylsubstituenten in 2-Stellung trägt.

Ein Trialkylammoniumphenylrest der Definition von $R^1$ erweist sich dann als besonders vorteilhaft, wenn der Phenylrest den Trialkylammoniumsubstituenten in 4-Stellung trägt. Die Alkylreste des Ammoniumsubstituenten entsprechen der gleichen Definition wie Alkyl in $R^1$ und können sowohl gleich als auch verschieden sein.

Der Biphenylylrest der Definition von $R^2$ ist vorteilhafterweise mit seiner 4-Stellung an das Tetrazoliumsalz geknüpft.

Der Naphthylrest in der Definition von $R^2$ ist vorteilhafterweise mit seiner 2-Position an das Tetrazoliumsalz gebunden.

Unter einem carboxylsubstituierten Hydroxyphenylrest in der Definition von $R^3$ wird vor allem ein solcher Hydroxyphenylrest verstanden, der in 2-Position einen Carboxylsubstituenten trägt und mit seiner 4-Position an das Tetrazoliumsalz gebunden ist.

Dimethylthiazolylreste in der Definition von $R^3$ sind mit ihrer 2-Position an das Tetrazoliumsalz gebunden. Ein bevorzugter Dimethylthiazolylrest trägt die Methylgruppen in 4- und 5-Position. Unter einem üblichen Gegenion in der Definition von $A^-$ werden vor allem einwertige Anionen verstanden. Üblicherweise werden hierzu anorganische Anionen, wie zum Beispiel Halogenionen verwendet. Bevorzugte Halogenionen in diesem Sinne sind Chlorid- und Bromidionen. Im Falle carboxylsubstituierter Reste $R^1$ oder $R^3$ kann das Gegenanion $A^-$ auch das Carboxylation sein.

Die in der folgenden Tabelle aufgelisteten Verbindungen sind besondere bevorzugt.

| Tetra zolium salz | $R^1$ | $R^2$ | $R^3$ |
|---|---|---|---|
| T1 | Carboxyl | Phenyl | 2-Carboxyphenyl |
| T2 | Phenyl | Phenyl | 2-Carboxyphenyl |
| T3 | 2,4-Dinitrophenyl | Phenyl | Phenyl |
| T4 | Carboxyl | Phenyl | Phenyl |
| T5 | para-Trimethylammoniumphenyl | Phenyl | Phenyl |
| T6 | 4-Carboxyphenyl | Phenyl | Phenyl |
| T7 | para-Nitrophenyl | para-Nitrophenyl | Phenyl |
| T8 | Phenyl | Phenyl | 4,5-Dimethylthiazolyl |
| T9 | Wasserstoff | Phenyl | Phenyl |
| T10 | n-Butyl | Phenyl | Phenyl |
| T11 | Phenyl | 2-Naphthyl | Phenyl |
| T12 | Phenyl | para-Diphenylyl | Phenyl |
| T13 | Methyl | Phenyl | 3-Carboxy-4-hydroxyphenyl |
| T14 | Methyl | Phenyl | Phenyl |
| T15 | Ethyl | Phenyl | Phenyl |
| T16 | 4-Carboxyphenyl | Phenyl | 2-Carboxyphenyl |
| T17 | 2-Carboxyphenyl | Phenyl | 2-Carboxyphenyl |
| Ganz besonders bevorzugte Verbindungen sind die Verbindungen T1, T2, T3, T4, T5, T6 und T8. | | | |

Die Konzentration der zur Entstörung der Meßreaktion zugesetzten Tetrazoliumsalze richtet sich nach der Menge der zu erwartenden störenden reduzierenden Substanz in der Probe. In der Regel werden 0,001 - 100 mmol/l, vorzugsweise 0,01 - 20 mmol/l, besonders bevorzugt 0,05 - 5 mmol/l Tetrazoliumsalz eingesetzt.

Das erfindungsgemäße Verfahren zur Bestimmung von Substraten oder Enzymaktivitäten unter Anwendung einer Redox-Reaktion als Meßreaktion und Zusatz eines oder mehrerer Tetrazoliumsalze zur Beseitigung störender reduzierender Substanzen kann je nach den für das jeweilige Verfahren erforderlichen Enzymen bei einem pH-Wert zwischen 3 und 11 durchgeführt werden. Bevorzugt ist der Bereich zwischen 6,5 und 9. Als Puffer kann jede Substanz verwendet werden, die in dem erforderlichen pH-Bereich eine ausreichende Pufferkapazität aufweist. Vorzugsweise werden jedoch Phosphat- oder Tris-Puffer verwendet. Die Konzentration des Puffers beträgt 0,01 bis 1 mol/l, vorzugsweise 0,05 bis 0,2 mmol/l.

Zur besseren Löslichkeit des aus dem zur Entstörung zugesetzten Tetrazoliumsalz gebildeten Formazans und zur Steigerung der Reaktionsgeschwindigkeit der entstörenden Reaktion kann der zu messenden Probe gegebenenfalls ein nichtionisches Detergens, wie zum Beispiel Triton X-100, Tween 80 oder Polyvinylpyrrolidon zugesetzt werden. Die Konzentrationen solcher gegebenenfalls zugesetzten Detergentien richten sich nach der kritischen Miceller Konzentration des jeweiligen Detergens. Sie liegen in der Regel zwischen 0,01 und 5 %, insbesondere zwischen 0,05 und 0,5 %.

In einer bevorzugten Ausführungsform des Verfahrens wird die entstörende Tetrazoliumsalz-Wirkung durch Zugabe eines oder mehrerer Elektronenüberträger beschleunigt. Zur Beschleunigung der Entstörreaktion und als Hilfsmittel bei der Oxidation von Störsubstanzen kann eine Reihe von Elektronenüberträgern verwendet werden. Als Beispiele seien genannt Phenazin-methosulfat, Phenazin-ethosulfat, 8-Dimethylamino-2,3-benzophenoxazin, 1-Methoxy-5-methylphenazinium-methylsulfat oder Diaphorase. Besonders bevorzugt sind hiervon Phenazin-ethosulfat oder Diaphorase.

Bei der Bestimmung von Substraten oder Enzymaktivitäten unter Verwendung einer Redox-Reaktion als Meßreaktion kann ein gewisser entstörender Effekt auch schon mit Elektronenüberträgern allein ohne zusätzliche Tetrazoliumsalze, festgestellt werden. Die Wirkung der vorgenannten Elektronenüberträger reicht jedoch für eine weitgehende oder gar völlige Entstörung einer Redox-Meßreaktion nicht aus. Hierzu ist die erfindungsgemäße Kombination mit einem Tetrazoliumsalz erforderlich.

Die der zu entstörenden Probe zuzusetzenden Konzentrationen der Elektronenüberträger sind vorzugsweise 0,005 - 100 mmol/l, besonders bevorzugt 0,05 - 5 mmol/l. Im Falle der Diaphorase werden vorzugsweise Mengen zwischen 0,001 und 100 U/ml eingesetzt, wobei der Bereich zwischen 0,1 und 20 U/ml besonders bevorzugt ist.

Ein weiterer Gegenstand der Erfindung ist ein Reagenz zur enzymatischen Bestimmung von Substanzen oder zur Bestimmung von Enzymaktivitäten, welches ein System zur Bestimmung eines Substrats oder Enzyms mit einer photometrisch detektierbaren Redoxreaktion als Meßreaktion und zusätzlich ein oder mehrere Tetrazoliumsalze enthält, die ebenso wie die bei einer Reduktion entstehenden entsprechenden Formazane nicht oder nur in vernachlässigbarem Maß bei der Wellenlänge der Meßreaktion Licht absorbieren. Die erfindungsgemäßen Tetrazoliumsalze beseitigen unter den in den betreffenden Substrat- oder Enzymtests vorherrschenden Bedingungen störende reduzierende Substanzen irreversibel, leicht und schnell und ermöglichen dadurch eine störungsfreie Messung.

Der für die Redox-Meßreaktion übliche Wellenlängenbereich liegt bei vielen in der klinischen Chemie wichtigen Substraten oder Enzymen zwischen 500 und 600 nm. Vor allem bewährt haben sich deshalb Tetrazoliumsalze der allgemeinen Formel I. Besonders bevorzugt sind in Kombination mit den übrigen zur Bestimmung erforderlichen Reagenzbestandteilen die Verbindungen T1 bis T17. Hiervon haben sich als ganz besonders vorteilhaft die Tetrazoliumsalze T1, T2, T3, T4, T5, T6 und T8 erwiesen.

Das erfindungsgemäße Reagenz enthält ein oder mehrere Tetrazoliumsalze, deren Konzentration sich nach der zu erwartenden Menge an störender, reduzierender Substanz richtet. In der Regel enthält es 0,001 - 100 mmol/l, vorzugsweise 0,01 - 20 mmol/l, besonders bevorzugt 0,05 - 5 mmol/l Tetrazoliumsalz.

Um bei der Bestimmung von Substraten oder Enzymaktivitäten unter Anwendung einer Redox-Reaktion als Meßreaktion und unter Zusatz eines oder mehrerer Tetrazoliumsalze zur Beseitigung störender reduzierender Substanzen je nach den erforderlichen Enzymen einen bestimmten pH-Wert einzuhalten, kann das erfindungsgemäße Reagenz einen Puffer enthalten. Der pH des erfindungsgemäßen Reagenz liegt in dem Bereich von pH 3 - 11, bevorzugterweise in dem Bereich von pH 6,5 - 9. Puffer, die einen solchen Wert einstellen können, sind prinzipiell alle diejenigen, die mit ihrem pK-Wert innerhalb des angegebenen pH-Bereichs liegen. Das erfindungsgemäße Reagenz kann jeden dieser Puffer enthalten. Als bevorzugte Puffer enthält das erfindungsgemäße Reagenz Phosphat- oder Tris-Puffer. Die Konzentration des Puffers im Reagenz kann 0,01 bis 1 mol/l betragen, vorzugsweise aber 0,05 bis 0,2 mol/l.

Zur besseren Löslichkeit des aus dem zur Entstörung zugesetzten Tetrazoliumsalz gebildeten Formazans und zur Steigerung der Reaktionsgeschwindigkeit der entstörenden Reaktion kann das erfindungsgemäße Reagenz gegebenenfalls auch ein nichtionisches Detergenz, wie z. B. Triton X-100, Tween 80 oder Polyvinylpyrrolidon enthalten. Die Konzentration solcher Detergentien richtet sich nach der kritischen Miceller Konzentration des jeweiligen Detergens. Sie liegt in der Regel zwischen 0,01 und 5 %, insbesondere zwischen 0,0 5 und 0,5 %.

Ein besonders bevorzugtes, erfindungsgemäßes Reagenz ist dadurch gekennzeichnet, daß es außer einem oder mehreren Tetrazoliumsalzen auch einen oder mehrere Elektronenüberträger enthält. Als solche kommen z. B. Phenazin-methosulfat, Phenazin-ethosulfat, 8-Dimethylamino-2,3-benzophenoxazin, 1-Methoxy-5-methylphenazinium-methylsulfat oder Diaphorase in Frage.

Als besonders geeignete Elektronenüberträger haben sich in Kombination mit den übrigen zur Bestimmung bestimmter Substrate oder Enzymaktivitäten erforderlichen Reagenzbestandteilen Phenazin-ethosulfat und/oder Diaphorase erwiesen.

Die Konzentration der Elektronenüberträger im Reagenz beträgt 0,005 - 100 mmol/l, vorzugsweise 0,05 - 5 mmol/l. Im Falle der Diaphorase enthält das Reagenz vorzugsweise 0,001 - 100 U/ml des Enzyms. Besonders bevorzugt ist der Konzentrationsbereich zwischen 0,1 und 20 U/ml.

Das erfindungsgemäße Reagenz kann als System zur Bestimmung eines Substrats oder Enzyms mit einer Redox-Reaktion als Meßreaktion, z. B.
zur Bestimmung von Glucose: Glucose-Oxidase, Peroxidase, 4-Aminophenazon und Phenol,
zur Bestimmung von Harnsäure: Uricase, Peroxidase, 2-Hydrazono-2,3-dihydro-3-methyl-6-sulfobenzothiazol und N-Ethyl-N-$\beta$-sulfoethyl-m-toluidin
oder zur Cholesterin-Bestimmung: Cholesterin-Esterase, Cholesterin-Oxidase, Peroxidase, 4-Aminophenazon und Phenol
enthalten.

Alle diese Systeme zur Bestimmung der genannten, beispielhaft angeführten Substrate sind bekannt. An ihrer Stelle können auch andere bekannte Systeme zur Bestimmung von Substraten oder Enzymen im Rahmen des erfindungsgemäßen Reagenz verwendet werden.

Als besonders wirkungsvoll hat sich das erfindungsgemäße Reagenz erwiesen, wenn es zur Beseitigung störender reduzierender Substanzen ein oder mehrere Tetrazoliumsalze und als Bestandteil des Systems zur Bestimmung eines Substrats oder Enzyms ein $H_2O_2$-bildendes Enzym, wie z. B. die obengenannte Glucose-Oxidase, Uricase oder Cholesterin-Oxidase enthält. Ebenso hat sich das erfindungsgemäße Reagenz als besonders vorteilhaft erwiesen, wenn es für die Meßreaktion ein Phenol, wie z. B. Phenol selbst oder 2,4,6-Tribromhydroxybenzoesäure oder ein Anilinderivat, wie z. B. N-Ethyl-N-$\beta$-sulfoethyl-m-toluidin enthält, welches mit Reagenzien, wie z. B. 4-Aminophenazon, 4-Amino-antipyrin oder 2-Hydrazono-2,3-dihydro-3-methyl-6-sulfobenzothiazol oxidativ gekuppelt werden kann. Beispiele für solche Redoxreaktionen als Meßreaktionen wurden für die Glucose-, Harnsäure- oder Cholesterin-Bestimmung bereits genannt.

Anwendbar ist die Erfindung auch auf dem Gebiet der Schnelldiagnostica. Derartige Schnelldiagnostica enthalten in der Regel die verschiedenen, für die Durchführung des Verfahrens benötigten Reagentien entweder in einem saugfähigen, unlöslichen Träger, wie Papier, Vlies, etc. imprägniert oder mit einem geeigneten Bindemittel auf einer Trägerfolie oder einem quellbaren Film als Überzug aufgebracht.

In einer Ausführungsform ist das erfindungsgemäße Reagenz, welches ein oder mehrere Tetrazoliumssalze zur Beseitigung störender reduzierender Substanzen enthält auf einem saugfähigen Träger, wie z. B. Papier, imprägniert. Auf diese Weise werden z. B. durch Ascorbinsäure praktisch nicht gestörte Testpapiere zum Nachweis von z. B. Glucose, Harnsäure oder Cholesterin erhalten.

Erfindungsgemäß kann Tetrazoliumsalz aber auch auf einen getrennten Träger aufgebracht werden, der mit dem Träger der übrigen Reagentien vereinigt, beispielsweise darübergelegt, verklebt oder gemeinsam eingesiegelt wird. In einer solchen Ausführungsform kann z. B. ein wasserlösliches Papier (z. B. gemäß DE-OS 24 36 598) mit Tetrazoliumsalz imprägniert werden, während das zur Bestimmung eines Substrats oder einer Enzymaktivität erforderliche System auf einem wasserunlöslichen, saugfähigen Trägermaterial imprägniert vorliegt. Beide Trägermaterialien werden vorteilhafterweise so aufeinander geschichtet, daß die zu bestimmende Probe zuerst auf das Tetrazoliumsalz trifft und dann auf das wasserunlösliche Trägermaterial.

In einer bevorzugten Ausführungsform können auch getrennte Zonen des Trägermaterials mit Tetrazoliumsalz(en) und Testreagentien imprägniert werden. In diesem Fall wird zweckmäßigerweise der Träger mit der zu untersuchenden Lösung derart in Berührung gebracht, daß die Lösung zuerst mit der Tetrazoliumsalzhaltigen Zone in Berührung kommt und von dort aus in die Zone gesaugt wird, welche die übrigen erforderlichen Testreagentien enthält. Zur Illustration einer solchen Ausführungsform wird in Abbildung 1 ein Beispiel für eine Vorrichtung im Querschnitt dargestellt, die es gemäß DE-A 30 29 579 erlaubt, einerseits aus Vollblut das für den Test erforderliche Serum bzw. Plasma abzutrennen und andererseits aufgrund eines speziell gestalteten Aufbaus der Reagenz- und Hilfsstoffschichten eine Temperierung, Vorreaktion und gezieltes Starten der Hauptreaktion erlaubt.

Die Vorrichtung gemäß Abbildung 1 ist wie folgt zusammengesetzt: Auf einer inerten Trägerfolie 8 ist ein aus Glasfasern bestehendes Transportvlies 7 befestigt. Diese Transportvlies 7 teilweise überdeckend ist ein ebenfalls aus Glasfasern bestehendes Vlies 5 mittels eines Fixiernetzes 6 befestigt. Zwischen Vlies 5 und Transportvlies 7 ist ein Entstörgewebe 4 angeordnet, welches mit Stoffen imprägniert ist, die in der Lage sind, die Meßreaktion störende reduzierende Substanzen zu beseitigen. Seitlich von Transportvlies 7 ist über eine Klebestelle 9 eine aus einem durchsichtigen Kunststoff bestehende transparente Folie 1 befestigt. Unter dieser transparenten Folie 1 ist eine Reagenzschicht 2 aufgebracht, die aus einem quell- oder saugfähigen Film besteht, in dem die für die Meßreaktion notwendigen Stoffe eingearbeitet sind. Unter der Reagenzschicht 2 ist eine Deckschicht 3 aufgebracht, welche üblicherweise aus einem Kunststoff- oder Gelatinefilm besteht, der mit stark reflektierenden Substanzen, wie Bariumsulfat, Titandioxid oder ähnlichen durchsetzt ist und als optisch weißer Hintergrund dient. Zur Beobachtung eingestrahltes Licht wird so vollständig remittiert und eventuelle Verfärbungen des Vlieses 7 sind nicht sichtbar. Reagenzschicht 2 und Deckschicht 3 werden gemeinsam als Testschicht bezeichnet.

Wird auf das Fixiernetz 6 Vollblut aufgetragen, dann wird dieses im Glasfaservlies 5 in Serum und Erythrozyten getrennt, wobei letztere festgehalten werden. Beim Durchtritt durch das Entstörgewebe 4 werden die Meßreaktion störende reduzierende Inhaltsstoffe beseitigt, so daß in den linken Bereich des Transportvlieses 7 nur Serum übertritt, welches keine die Messung störende Substanzen enthält. Nach Druck auf die transparente Folie 1 wird die Meßreaktion gestartet, nachdem das Serum das Transportvlies 7 vollständig gefüllt hat. Das Serum dringt infolge des Druckkontaktes durch die Deckschicht 3 in die Reagenzschicht 2 und durchfeuchtet diese gleichmäßig. Die Reaktion wird anhand der Verfärbung in der Reagenzschicht 2 durch die transparente Folie 1 hindurch beobachtet.

Die für das erfindungsgemäße Verfahren und Reagenz verwendbaren Tetrazoliumsalze 5-Carboxy-3-(2-carboxyphenyl)-2-phenyl-2-H-tetrazoliumhydroxid, inneres Salz (T1),
3-(2-Carboxyphenyl)-2.5-diphenyl-2-H-tetrazoliumhydroxid, inneres Salz (T2),
2,3-Diphenyl-5-(2.4-dinitrophenyl)-2-H-tetrazoliumchlorid (T3), 2.3-Diphenyl-5-[4-(trimethylammoniophenyl)]-2-H-tetrazolium-dichlorid (T5) sowie
3-(2-Carboxyphenyl)-5-(4-carboxyphenyl)-2-phenyl-2-H-tetrazoliumhydroxid, inneres Salz (T16) und
3,5-Bis-(2-carboxyphenyl)-2-phenyl-2-H-tetrazoliumchlorid (T17) sind neue Verbindungen und ebenfalls Gegenstand der Erfindung. Sie können durch die allgemeine Formel I′

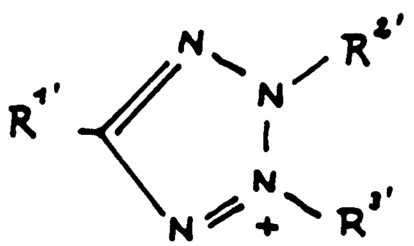

(I')

in der

- $R^{1'}$ einen Carboxylrest, einen unsubstituierten oder einen in 2- oder 4-Stellung durch eine Carboxylgruppe, in 2-und 4-Stellung durch Nitrogruppen disubstituierten oder in 4-Stellung durch Trimethylammonium substituierten Phenylrest,
- $R^{2'}$ einen unsubstituierten Phenylrest,
- $R^{3'}$ einen unsubstituierten oder in 2-Stellung durch eine Carboxylgruppe substituierten Phenylrest und
- $A'^{-}$ ein übliches Gegenanion

bedeuten,
beschrieben werden.

Die üblichen Gegenanionen in der Definition von $A'^{-}$ entsprechen denjenigen, die für $A^{-}$ in der allgemeinen Formel I angegeben wurden.

Außer ihrer guten Wasserlöslichkeit zeigen die Verbindungen anforderungsgemäß keine Lichtabsorption im Wellenlängenbereich zwischen 500 und 600 nm und stören die Meßreaktion nicht.

Ihre Herstellung erfolgt nach an sich bekannten Methoden, die beschrieben sind bei:
R. Kuhn, D. Jerchel, Ber. Dtsch. Chem. Ges.74, 94 (1941);
D. Jerchel, W. Möhle, Ber. Dtsch. Chem. Ges. 77, 600 (1944);
R. Wizinger, V. Bisro, Helv. Chim. Acta 32, 909 (1949).

Hierbei werden literaturbekannte Aldehydphenylhydrazone der allgemeinen Formel II

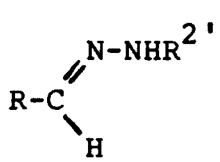

(II)

in der

- R einen veresterten Carboxylrest, einen unsubstituierten Phenylrest oder einen in 2- oder 4-Stellung durch eine Carboxylgruppe, in 2- und 4-Stellung durch Nitrogruppen disubstituierten oder in 4-Stellung durch Trimethylammonium substituierten Phenylrest und
- $R^{2'}$ einen unsubstituierten Phenylrest bedeuten,

mit Diazoniumsalzen der allgemeinen Formel III

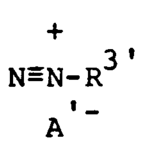

(III)

in der

$R^{3'}$ einen unsubstituierten oder in 2-Stellung durch eine Carboxylgruppe substituierten Phenylrest und

$A'^{-}$ ein übliches Gegenanion

bedeuten,

durch alkalische Kupplung in Formazane der allgemeinen Formel IV

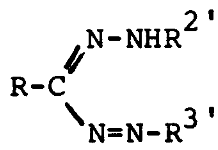

(IV)

in der

R und $R^{2'}$ die in der allgemeinen Formel II angegebene Bedeutung haben und

$R^{3'}$ der Definition in der allgemeinen Formel III entspricht,

überführt.

Ein veresterter Carboxylrest in der Definition von R ist hierbei vorzugsweise ein niederalkylveresterter Carboxylrest, wobei Niederalkyl eine $C_1$ bis $C_4$-Kohlenwasserstoffrest bedeutet. Besonders bevorzugt sind Methyl- oder Ethylester.

Übliche Gegenanionen der Definition von $A'^{-}$ sind die gleichen wie für $A^{-}$ in der allgemeinen Formel I angegeben.

Als basisches Reaktionsmedium für die alkalische Azokupplung können z. B. Pyridin, Natriumacetat gelöst in Dimethylformamid und/oder Alkohol oder alkoholische Alkalilauge Verwendung finden.

Unter Alkohol werden niedere aliphatische Alkohole, bevorzugt Methanol oder Ethanol verstanden.

Die Überführung von Formazanen der allgemeinen Formel IV in Tetrazoliumsalze der allgemeinen Formel I′ gelingt durch Oxidation entweder mittels Isopentylnitrit in alkoholischer Salzsäure analog D. Jerchel, H. Fischer, Liebigs Ann. Chem. 563, 200 (1949) oder mittels Bleitetraacetat analog R. Kuhn, D. Jerchel, Ber. Dtsch. Chem. Ges. 74, 941 (1941) oder D. Jerchel, H. Fischer, Liebigs Ann. Chem. 563, 200 (1949) in Chloroform.

Die Amylnitrit-Methode hat hierbei den Vorteil, daß eine aufewendige, säulenchromatographische Nachreinigung entfallen kann. Sie ist jedoch wegen des geringen Oxidationspotentials des Nitrits nicht universell anwendbar.

Da die Doppelbindungen und die positive Ladung des Tetrazoliumringes innerhalb des Stickstoffsystems nicht lokalisierbar sind, kann zur Herstellung der erfindungsgemäßen Verbindungen der allgemeinen Formel I′ natürlich auch analog von Formazanen der allgemeinen Formel IV′

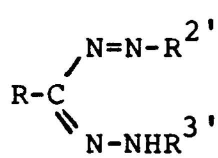

(IV')

in der

R, $R^{2'}$ und $R^{3'}$ die gleiche Bedeutung haben wie in der Definition der allgemeinen Formel IV, ausgegangen werden.

Formazane der allgemeinen Formel IV′ sind dem geschilderten Verfahren analog aus Aldehydphenylhydrazonen der allgemeinen Formel II′

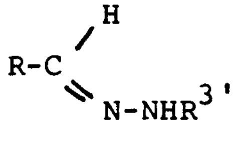

(II')

und Diazoniumsalzen der allgemeinen Formel III'

$$N\equiv\overset{+}{N}-R^{2'} \quad A'^{-} \qquad (III')$$

herstellbar. Die angegebenen Reste haben die gleiche Bedeutung wie die in den allgemeinen Formeln II und III definierten.

Die folgenden Beispiele erläutern die Erfindung weiter.

Beispiel 1

Herstellung der Tetrazoliumsalze T1, T2, T3, T5, T16 und T17

a) Herstellung der Ausgangsformazane

0,1 mol Amin werden in 100 ml Wasser suspendiert und nach Zugabe von 0,3 mol 12 N Salzsäure durch Zutropfen einer konzentrierten wässrigen Lösung von 1,04 mol Natriumnitrit bie 0-5° C diazotiert. Innerhalb 30 Minuten wird die gekühlte Diazoniumsalzlösung unter Rühren und Kühlung unterhalb 10° C in eine Lösung von 0,1 mol Aldehydphenylhydrazon und 0,35 mol Natriumacetat (oder 100 ml Pyridin) in 180 ml Ethanol und 100 ml Dimethylformamid eingetropft und eine Stunde nachgerührt. Der gebildete Kristallbrei wird abgesaugt, gut mit Wasser und danach mit wenig Methanol gewaschen und abschließend getrocknet. Eine Reinigung des so erhaltenen Formazans kann durch Umkristallisieren aus Eisessig, Methanol-Wasser oder aus Dimethylformamid-Wasser oder durch Säulenchromatographie an Kieselgel 60 (Merck) mit Methylenchlorid-Methanol 5:1 oder Methylenchlorid als Eluens erfolgen. Nach dieser Methode wurden die folgenden Formazane erhalten.

| Formazan | Ausgangs-verbindung für | Ausbeute (%) | Fp (° C) | Azokupplung in Medium | Reinigungsmethode |
|---|---|---|---|---|---|
| F1 | T1 | 40 | 180 (Zers.) | A | Umkristallisation aus Methanol/ Wasser |
| F2 | T2 | 40 | 187 (Zers.) | C | R. Wizinger V. Bisro, Helv. Chim. Acta 32, 910 (1949) |
| F3 | T3 | 62 | 204 (Zers.) | B | Säulenchromatographie an Kieselgel, Eluens: Methylenchlorid |
| F5 | T5 | 70 | 187 (Zers.) | A | Anrühren mit Isopropanol |
| F16 | T16 | 35 | >300 (Zers.) | B | Säulenchromatographie an Kieselgel, Eluens: Methylenchlorid-Methanol = 5:1 |
| F17 | T17 | 50 | 191 (Zers.) | A | Säulenchromatographie an Kieselgel, Eluens: Methylenchlorid-Methanol = 5:1 |

Medium A: Dimethylformamid/Natriumacetat
B: Dimethylformamid/Pyridin
C: Ethanolische Lauge (KOH oder NaOH)
F1 2-(3-Carboxyethyl-5-phenyl-1-formazano)benzoesäure
F2 2-(3.5-Diphenyl-1-formazano)benzoesäure
F3 1.5-Diphenyl-3-(2.4-dinitrophenyl)formazan
F5 4-(1.5-Diphenyl-3-formazano)-N,N,N-trimethylammoniochlorid
F16 2,4'-(5-Phenyl-1.3-formazandilyl)bis-benzoesäure
F17 2,2'-(5-Phenyl-1,3-formazandilyl)bis-benzoesäure

b) Oxidation der Formazane

A) Bleitetraacetat-Methode

0,1 mol Formazan werden in wasserfreiem Chloroform gelöst und mit 0,12 mol Bleitetraacetat versetzt. Man läßt 30 Minuten rühren und saugt danach von Ungelöstem ab. Das Filtrat wird eingedampft, der Rückstand nach Zugabe von Wasser abgesaugt und das Filtrat mit 2 N Salzsäure angesäuert. Das so gefällte Bleichlorid wird abgesaugt, das Filtrat eingeengt und der Rückstand aus Ethanol umkristallisiert oder durch Säulenchromatographie an Kieselgel 60 (Merck) mit Chloroform-Methanol 19:1, Chloroform-Methylenchlorid 5:1 oder Methylenchlorid-Methanol 5:1 als Eluens gereinigt.

B) Isopentylnitrit-Methode

0,1 mol Formazan werden in der 10- bis 20fachen Menge Ethanol suspendiert, 0,2 mol Isopentylnitrit zugegeben und innerhalb 20 Minuten 0,15 mol ethanolische Salzsäure unter Rühren zugetropft. Anschließend wird eine Stunde nachgerührt. Falls dann die Reaktionsmischung noch nicht entfärbt ist, wird sie bis zur vollständigen Entfärbung auf 40° C erwärmt. Tetrazoliumsalz wird durch Zugabe von Äther ausgefällt, abgesaugt und getrocknet. Zur Reinigung kann das Produkt mit Methanol oder Isopropanol angerührt, abgesaugt und getrocknet werden. Umkristallisation kann aus Methanol, Methanol-Wasser oder Eisessig erfolgen. Vorteilhaft läßt sich zur Reinigung der Tetrazoliumsalze die Säulenchromatographie an Kieselgel 60 (Merck) mit Chloroform-Methanol 19:1, Chloroform-Methylenchlorid 5:1 oder Methylenchlorid-Methanol = 5:1 anwenden.

Nach einer der beiden Methoden A) oder B) wurden die folgenden Tetrazoliumsalze hergestellt.

| Tetrazoliumsalz | Ausbeute (%) | Fp (° C) | Oxidations-methode | Reinigungs-methode |
|---|---|---|---|---|
| T1 | 35 | 184 (Zers.) | A | Säulenchromatographie an Kieselgel, Eluens: Chloroform/Methanol |
| T2 | 48 | 259 (Zers.) | A | Umkristallisation aus Ethanol-Wasser |
| T3 | 47 | 246 (Zers.) | A | Säulenchromatographie an Kieselgel, Eluens: Methylenchlorid-Methanol = 5:1 |
| T5 | 82 | 162 (Zers.) | A | Anrühren mit Methanol |
| T16 | 42 | >300 (Zers.) | A | Säulenchromatographie an Kieselgel, Eluens: Methylenchlorid/ Methanol = 5:1 |
| T17 | 74 | 230 (Zers.) | B | Säulenchromatographie an Kieselgel, Eluens: Methylenchlorid/ Methanol = 5:1 |

Oxidations-Methode A: Bleitetraacetat-Chloroform

B: Isopentylnitrit-ethanolische Salzsäure

T1 5-Carboxy-3-(2-carboxyphenyl)-2-phenyl-2-H-tetrazolium hydroxid, inneres Salz

T2 3-(2-Carboxyphenyl)-2.5-diphenyl-2-H-tetrazoliumhydroxid, inneres Salz

T3 2,3-Diphenyl-5-(2.4-dinitrophenyl)-2-H-tetrazoliumchlorid

T5 2.3-Diphenyl-5-[4-(trimethylammoniophenyl)]-2-H-tetrazolium-dichlorid

T16 3-(2-Carboxyphenyl)-5-(4-carboxyphenyl)-2-phenyl-2-H-tetrazoliumhydroxid, inneres Salz

T17 3.5-Bis-(2-carboxyphenyl)-2-phenyl-2-H-tetrazoliumchlorid

Beispiel 2

Harnsäurebestimmung in Gegenwart verschiedener Störsubstanzen

In die Küvetten A, B und C der Schichtdicke 1 cm werden die folgenden Lösungen pipettiert:

| | A | B | C |
|---|---|---|---|
| Probe | 50 µl | 50 µl | 50 µl |
| Störsubstanz * | - | + | + |
| Tetrazoliumsalz und/oder * Elektronenüberträger | - | - | 175 µl |
| 0,1 M Kaliumphosphat-Puffer pH 8 | 175 µl | 175 µl | - |

* Art und Konzentration sind in den Beispielen 2 a) - h) jeweils angegeben.

Die in die jeweiligen Küvetten pipettierten Lösungen werden gemischt und eine Minute bei 25° C inkubiert. Dann erfolgt die Zugabe von 2 ml Harnsäurereagenz der folgenden Zusammensetzung:
Kaliumphosphat 0,1 mol/l, pH 8
2,4,6-Tribromhydroxybenzoesäure 20 mmol/l
4-Aminoantipyrin 0,1 mmol/l
Natriumazid 1 g/l
Peroxidase 4 U/ml
Uricase 2 U/ml
Nach 5minütiger Inkubation bei 25° C wird die Extinktion der jeweiligen Reaktionsmischung bei einer Wellenlänge von 546 nm gegen einen Reagenzienleerwert (ohne Probe) gemessen. Zur Kalibrierung wird statt einer zu messenden Probe ein wässriger Harnsäure-Standard (6 mg/dl) verwendet.

Die gemessene Harnsäurekonzentration in Küvette A (ohne Störsubstanz) wird als 100 % eingesetzt. Die Harnsäurewiederfindungsrate in Anwesenheit von Störsubstanz wird in den folgenden Beispielen 2 a) bis 2 h) experimentell bestimmet. Die Ergebnisse belegen, daß die Wiederfindungsrate in Küvettenreihe C (mit Tetrazoliumsalzzusatz) deutlich höher ist als ohne Zusatz (Küvettenreihe B).

Die im folgenden angegebene Konzentration der Tetrazolium-Salze und/oder Elektronenüberträger betreffen die Endkonzentration in der zu messenden Lösung nach Zugabe dieser Substanzen und von Puffer. Die Konzentration der Störsubstanzen betrifft die Konzentration in der Probe vor Zugabe von Puffer, Tetrazoliumsalz und/oder Elektronenüberträger.

a) Störsubstanz: Ascorbat 3 mg/dl
T3: 0,4 mmol/l
Phenazin-ethosulfat (PES): 20 µmol/l

| | % Wiederfindung in | |
|---|---|---|
| | B | C |
| Humanserum 1 | 61 | 100 |
| Humanserum 2 | 65 | 100 |
| Humanserum 3 | 62 | 92 |
| Humanserum 4 | 58 | 110 |
| Humanserum 5 | 83 | 105 |
| Kontrollserum PPU Ch. 1 | 80 | 101 |
| Kontrollserum PPU Ch. 2 | 74 | 104 |

b) Störsubstanz: Bilirubin
T8: 0,4 mmol/l
PES: 20 µmol/l

| Bilirubin Konzentration in der Probe (Humanserum) | % Wiederfindung in | |
|---|---|---|
| | B | C |
| 100 mg/1<br>200 mg/l | 76<br>63 | 89<br>86 |

c) Störsubstanz: ∝-Methyl-DOPA 20 mg/1
T3 bzw. T5: 0,6 mmol/l
PES: 25 µmol/l
Probe: Harnsäure-Standard 6 mg/dl

| Tetrazoliumsalz | % Wiederfindung in | |
|---|---|---|
| | B | C |
| T3<br>T5 | 62<br>62 | 94<br>94 |

d) Störsubstanz: Calcium-Dobesylat 25 mg/l
T3: 0,6 mmol/l
PES: 25 µmol/l

| % Wiederfindung in | |
|---|---|
| B | C |
| 90 | 98 |

e) Störsubstanz: Ascorbat 3 mg/dl
Tetrazoliumsalz: 0,06 mmol/l
PES: 25 µmol/l
Probe: Harnsäure-Standard 6 mg/dl

| Tetrazoliumsalz | % Wiederfindung in | |
|---|---|---|
| | B | C |
| T1<br>T2<br>T3<br>T4<br>T5<br>T6 | 39<br>39<br>39<br>39<br>39<br>39 | 82<br>88<br>82<br>89<br>94<br>93 |

f) Störsubstanz: Ascorbat 3 mg/dl
T8: 0,4 mmol/l
PES: 20 µmol/l
Probe: Humanserum

| % Wiederfindung in | |
|---|---|
| B | C |
| 72 | 93 |

g) Störsubstanz: Ascorbat 3 mg/dl
T8: 0,4 mmol/l

Diaphorase 0,4 U/ml

| Probe: Humanserum | % Wiederfindung in | |
|---|---|---|
| | B | C |
| | 65 | 96 |

h) Störsubstanz: Ascorbat 17,6 mg/dl
PES: 25 µmol/l
Probe: Harnsäure Standard 6 mg/dl

| % Wiederfindung in | |
|---|---|
| B | C |
| 6 | 53 |

Beispiel 3

Glucosebestimmung in Gegenwart von Ascorbinsäure

In die Küvetten A, B und C der Schichtdicke 1 cm werden die folgenden Lösungen pipettiert:

| | A | B | C |
|---|---|---|---|
| Probe<br>Ascorbinsäure | 50 µl<br>- | 50 µl<br>+ | 50 µl<br>+ |
| Tetrazoliumsalz: T3 und Elektronenüberträger: Phenazin-ethosulfat (PES) | - | - | 175 µl |
| 0,1 M Kaliumphosphat-Puffer pH 7,8 | 175 µl | 175 µl | - |

Die in die jeweiligen Küvetten pipettierten Lösungen werden gemischt und fünf Minuten bei 25° C inkubiert. Dann erfolgt die Zugabe von 2 ml Glucosereagenz der folgenden Zusammensetzung:

| | |
|---|---|
| Tris-Phosphat-Puffer | 180 mmol/l, pH 7,8 |
| Phenol | 11 mmol/l |
| 3,4-Dichlorphenol | 2,1 mmol/l |
| Fettalkoholpolyglycoläther | 0,24 % |
| 4-Aminophenazon | 0,8 mmol/l |
| Peroxidase | 1 U/ml |
| Glucoseoxidase | 15 U/ml |

Nach 30minütiger Inkubation bei 25° C wird die Extinktion der jeweiligen Reaktionsmischung bei einer Wellenlänge von 546 nm gegen einen Reagentienleerwert (ohne Probe) gemessen. Zur Kalibrierung wird statt einer zu messenden Probe ein wässriger Glucose-Standard. (100 mg/dl) verwendet.

Die gemessene Glucosekonzentration in Küvette A (ohne Störsubstanz) wird als 100 % eingesetzt. Die Glucosewiederfindungsrate in Anwesenheit von Störsubstanz wird in den folgenden Beispielen 3 a) und 3 b) experimentell bestimmt. Die Ergebnisse belegen, daß die Wiederfindungsrate in Küvettenreihe C (mit Tetrazoliumsalzzusatz) deutlich höher ist als ohne Zusatz (Küvettenreihe B).

Die im folgenden angegebene Konzentration des Tetrazoliumsalzes T3 und des Elektronenüberträgers PES betreffen die Endkonzentration in der zu messenden Lösung nach Zugabe dieser Substanzen und von Puffer. Die Konzentration der Störsubstanz Ascorbinsäure betrifft jeweils die Konzentration in der Probe vor

Zugabe von Puffer, T3 und PES.

a) Glucose-Konzentration: 25 mg/dl
Störsubstanz: Ascorbat
T3: 0,25 mmol/l
PES: 20 µmol/l

| Ascorbat-Konzentration | % Wiederfindung in | |
|---|---|---|
| | B | C |
| 3 mg/dl | 65 | 94 |
| 6 mg/dl | 27 | 91 |
| 12 mg/dl | 0 | 85 |

b) Glucose-Konzentration: 50 mg/dl
Störsubstanz: Ascorbat 3 mg/dl
T3: 0,25 mmol/l
PES: 20 µmol/l

| % Wiederfindung in | |
|---|---|
| B | C |
| 83 | 94 |

Beispiel 4

Testsystem zur Bestimmung von Harnsäure in Blut

a) Testschicht zum Nachweis der Harnsäure

Aus den unten aufgeführten Bestandteilen wird eine Beschichtungsmasse hergestellt und mit einer Naßfilmdicke von 200 µm auf eine transparente Folie 1 aufgerakelt und getrocknet: 18 g einer Kunststoffdispersion eines Mischpolymeren aus Vinylacetat und Vinylpropionat; 1,38 g Alginat; 69 g eines Tris-Citrat-Puffers pH 7,5 0,45 m; 0,47 g Indikator 2-(3,5-Dimethoxy-4-hydroxyphenyl)-4-(5)-(4-dimethylaminophenyl)-5-(4)-methyl-(1H)-imidazol-hydrochlorid; 0,025 g 1-(3-Chlorphenyl)-semicarbazid; 0,025 g $MgK_2EDTA.2H_2O$; 0,5 g Triton X 100; 0,6 g Hexanol; 200 KU Peroxidase; 2 KU Uricase.

Auf die so hergestellte Reagenzschicht 2 wird eine Deckschicht 3 als optisch weißer Hintergrund der unten aufgeführten Zusammensetzung mit einer Schichtdicke von 200 µm gerakelt und getrocknet: 52 ml 0,1 m Tris-Citrat-Puffer pH 7,0; 5,5 g Titandioxid; 2,7 g Diatomeenerde; 0,4 g Alginat; 1,4 g einer Kunststoffdispersion eines Mischpolymeren aus Vinylacetat und Vinylpropionat; 0,2 g Triton X 100.

b) Entstörgewebe 4

Nylon-Gewebe (Type NY 75 HC der Firma Züricher Beuteltuchfabrik, Schweiz) wird mit folgender Lösung getränkt und bei 60° C getrocknet:
5 mg 5-(2,4-Dinitrophenyl)-2,3-diphenyl-tetrazolium-chlorid (T3); 5 mg Phenazin-ethosulfat; 30 mg Dioctylsulfosuccinat-Natrium; ad 100 ml 0,5 m Phosphatpuffer pH 7,5.

Als Material für einen Vergleichstest wurde das gleiche Gewebe mit einer entsprechenden Lösung getränkt, die nur Puffer und Netzmittel, aber kein Tetrazoliumsalz und Phenazin-ethosulfat enthielt.

c) Gesamtsystem

Die vorgenannten Bestandteile werden zu einem Testsystem gemäß Abbildung 1 verarbeitet. Der Test ist zur Bestimmung der Harnsäure aus Blut, Plasma und Serum geeignet.

d) Testdurchführung

Zur Bestimmung der Harnsäure werden 30 µl Serum auf das Fixiernetz 6 aufgetragen, die transparente Folie 1 wird nach einer Minute angedrückt und nach weiteren zwei Minuten wird mit einem Remissionsphotometer die gebildete Farbe vermessen und aus einer vorher ermittelten Eichkurve die Harnsäurewerte ermittelt.

Eine Serumprobe mit einem Harnsäuregehalt von 6,2 mg/dl wird in zwei Teile geteilt. Einem Teil wird Ascorbinsäure zugefügt, so daß ein Gehalt von 2 mg/dl resultiert. Beide Serumproben werden sowohl mit dem Tetrazoliumsalz enthaltenden Testsystem als auch mit dem zum Vergleich dienenden Testsystem ohne Tetrazoliumsalz untersucht.

Es werden folgende Harnsäure-Werte in mg/dl gemessen:

| | Serum ohne Ascorbinsäure | Serum mit Ascorbinsäure |
|---|---|---|
| mit Tetrazoliumsalz | 6,27 | 6,09 |
| ohne Tetrazoliumsalz | 6,02 | 4,61 |

Bezugszeichenliste:

1 = Transparente Folie
2 = Reagenzschicht
3 = Deckschicht
4 = Entstörgewebe
5 = Glasfaservlies
6 = Fixiernetz
7 = Transportvlies
8 = Trägerfolie
9 = Klebestelle

## Patentansprüche

1. Verfahren zur Bestimmung von Substraten oder Enzymaktivitäten unter Anwendung einer photometrisch detektierbaren Redoxreaktion als Meßreaktion, dadurch gekennzeichnet, daß in Gegenwart eines oder mehrerer zusätzlich zugegebener Tetrazoliumsalze gearbeitet wird, wobei die Tetrazoliumsalze und deren Formazane nicht oder nur in vernachlässigbarem Maß bei der Wellenlänge der Meßreaktion Licht absorbieren.

2. Verfahren gemäß Anspruch 1, dadurch gekennzeichnet, daß als zusätzliche Tetrazoliumsalze solche der allgemeinen Formel I

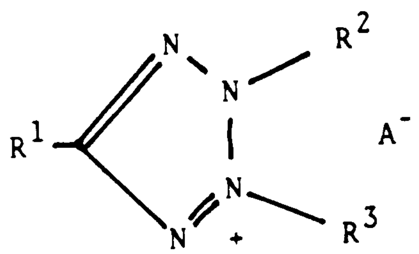

(I)

verwendet werden,
in der
$R^1$ Wasserstoff, einen Carboxyl-, Alkyl-, Phenyl-, Nitrophenyl-, Dinitrophenyl-, carboxylsubstituierten Phenyl- oder einen Trialkylammoniumphenylrest,
$R^2$ einen Phenyl-, Nitrophenyl-, Biphenylyl- oder Naphthylrest,
$R^3$ einen Phenyl-, carboxylsubstituierten Phenyl-, einen carboxylsubstituierten Hydroxyphenyl- oder Dimethylthiazolylrest und
$A^-$ ein übliches Gegenion
bedeuten.

3. Verfahren gemäß einem der Ansprüche 1 bis 2, dadurch gekennzeichnet, daß außerdem ein oder mehrere Elektronenüberträger eingesetzt werden.

4. Verfahren gemäß Anspruch 3, dadurch gekennzeichnet, daß als Elektronenüberträger Phenazinmethosulfat, Phenazinethosulfat, 8-Dimethylamino-2,3-benzophenoxazin, 1-Methoxy-5-methylphenazinium-methylsulfat oder Diaphorase verwendet werden.

5. Reagenz zur enzymatischen Bestimmung von Substraten oder zur bestimmung von Enzymaktivitäten, enthaltend ein System zur Bestimmung eines Substrates oder Enzyms mit einer photometrisch detektierbaren Redox-Reaktion als Meßreaktion, dadurch gekennzeichnet, daß es zusätzlich ein oder mehrere Tetrazoliumsalze enthält, die ebenso wie die bei einer Reduktion entstehenden entsprechenden Formazane nicht oder nur in vernnachlässigbarem Maß bei der Wellenlänge der Meßreaktion Licht absorbieren.

6. Reagenz gemäß Anspruch 5, dadurch gekennzeichnet, daß es als zusätzliche Tetrazoliumsalze solche der allgemeinen Formel I

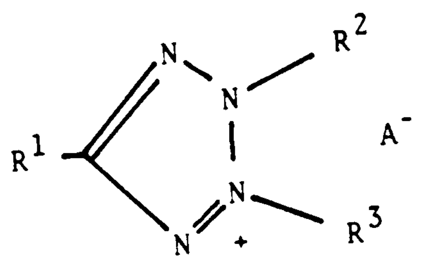

(I)

enthält,
in der

$R^1$ Wasserstoff, einen Carboxyl-, Alkyl-, Phenyl-, Nitrophenyl-, Dinitrophenyl-, carboxylsubstituierten Phenyl- oder einen Trialkylammoniumphenylrest,
$R^2$ einen Phenyl-, Nitrophenyl-, Biphenylyl- oder Naphthylrest,
$R^3$ einen Phenyl-, carboxylsubstituierten Phenyl-, carboxylsubstituierten Hydroxyphenyl-oder einen Dimethylthiazolylrest und
$A^-$ ein übliches Gegenion

bedeuten.

7. Reagenz gemäß einem der vorgenannten Ansprüche, dadurch gekennzeichnet, daß es zusätzlich einen oder mehrere Elektronenüberträger enthält.

8. Reagenz gemäß einem der Ansprüche 5 bis 7, dadurch gekennzeichnet, daß es auf einem unlöslichen Träger imprägniert vorliegt.

9. Tetrazoliumsalze der allgemeinen Formel I′

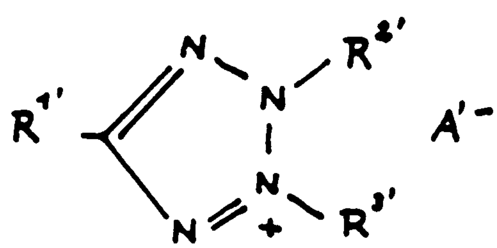

(I')

in der

$R^{1'}$ einen Carboxylrest, einen unsubstituierten oder einen in 2- oder 4-Stellung durch eine

Carboxylgruppe, in 2-und 4-Stellung durch Nitrogruppen disubstituierten oder in 4-Stellung durch Trimethylammonium substituierten Phenylrest,

$R^{2'}$ einen unsubstituierten Phenylrest,

$R^{3'}$ einen unsubstituierten oder in 2-Stellung durch eine Carboxylgruppe substituierten Phenylrest und

$A'^{-}$ ein übliches Gegenanion

bedeuten.

**10.** Verfahren zur Herstellung von Verbindungen der allgemeinen Formel I'

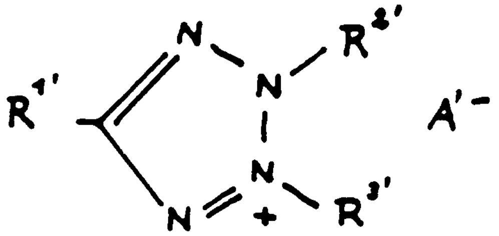

(I')

in der

$R^{1'}$ einen Carboxylrest, einen unsubstituierten oder einen in 2- oder 4-Stellung durch eine Carboxylgruppe, in 2-und 4-Stellung durch Nitrogruppen disubstituierten oder in 4-Stellung durch Trimethylammonium substituierten Phenylrest,

$R^{2'}$ einen unsbustituierten Phenylrest,

$R^{3'}$ einen unsubstituierten oder in 2-Stellung durch eine Carboxylgruppe substituierten Phenylrest und

$A'^{-}$ ein übliches Gegenanion

bedeuten,

dadurch gekennzeichnet, daß entweder

a) Aldehydphenylhydrazone der allgemeinen Formel II

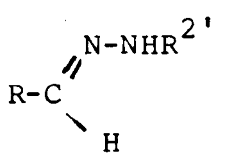

(II)

in der

R einen veresterten Carboxylrest, einen unsubstituierten Phenylrest oder einen in 2- oder 4-Stellung durch eine Carboxylgruppe, in 2- und 4-Stellung durch Nitrogruppen disubstituierten oder in 4-Stellung durch Trimethylammonium substituierten Phenylrest und

$R^{2'}$ einen unsubstituierten Phenylrest

bedeuten,

mit Diazoniumsalzen der allgemeinen Formel III

$$\overset{+}{N}\equiv N-R^{3'} \quad A'^{-} \qquad \text{(III)}$$

in der

$R^{3'}$ einen unsubstituierten oder in 2-Stellung durch eine Carboxylgruppe substituierten Phenylrest und

$A'^{-}$ ein übliches Gegenanion

bedeuten,

alkalisch zu Formazanen der allgemeinen Formel IV

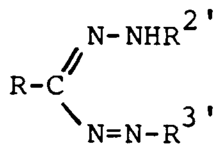

(IV)

in der
R, $R^{2'}$ und $R^{3'}$ die vorgenannte Bedeutung haben, gekuppelt oder
b) Aldehydphenylhydrazone der allgemeinen Formel II′

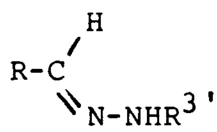

(II')

in der

- R einen veresterten Carboxylrest, einen unsubstituierten Phenylrest oder einen in 2- oder 4-Stellung durch eine Carboxylgruppe, in 2- und 4-Stellung durch Nitrogruppen disubstituierten oder in 4-Stellung durch Trimethylammonium substituierten Phenylrest und
- $R^{3'}$ einen unsubstituierten oder in 2-Stellung durch eine Carboxylgruppe substituierten Phenylrest

bedeuten,
mit Diazoniumsalzen der allgemeinen Formel III'

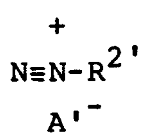

(III')

in der

- $R^{2'}$ einen unsubstituierten Phenylrest und
- $A'^{-}$ ein übliches Gegenanion

bedeuten,
alkalisch zu Formazanen der allgemeinen Formel IV'

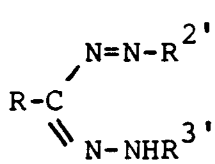

(IV')

in der R, $R^{2'}$ und $R^{3'}$ die vorgenannte Bedeutung haben, gekuppelt und diese abschließend oxidiert werden.

**Claims**

1. Process for the determination of substrates or of enzyme activities with the use of a photometrically detectable redox reaction as measurement reaction, characterised in that one operates in the presence of one or more additionally added tetrazolium salts, whereby the tetrazolium salts and their formazanes do not absorb light or only in a negligible amount at the wavelength of the measurement reaction.

2. Process according to claim 1, characterised in that, as additional tetrazolium salts, there are used those

of the general formula I

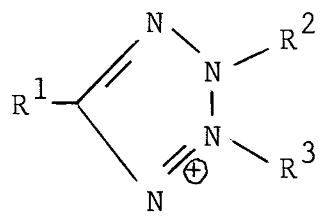 $A^{\ominus}$ (I)

in which $R^1$ signifies hydrogen, a carboxyl, alkyl, phenyl, nitrophenyl, dinitrophenyl, carboxyl-substituted phenyl or trialkylammoniumphenyl radical, $R^2$ a phenyl, nitrophenyl, biphenylyl or naphthyl radical, $R^3$ a phenyl, carboxyl-substituted phenyl, a carboxy-substituted hydroxyphenyl or dimethylthiazolyl radical and $A^{\ominus}$ a conventional counterion.

**3.** Process according to claim 1 or 2, characterised in that, in addition, one or more electron transmitters are used.

**4.** Process according to claim 3, characterised in that, as electron transmitters, there are used phenazine-methosulphate, phenazine-ethosulphate, 8-dimethylamino-2,3-benzophenoxazine, 1-methoxy-5-methylphenazinium-methyl sulphate or diaphorase.

**5.** Reagent for the enzymatic determination of substrates or for the determination of enzyme activities, containing a system for the determination of a substrate or enzyme with a photometrically detectable redox reaction as measurement reaction, characterised in that it additionally contains one or more tetrazolium salts which, in the same way as the corresponding formazanes resulting in the case of a reduction, do not absorb light or only in a negligible amount at the wavelength of the measurement reaction.

**6.** Reagent according to claim 5, characterised in that, as additional tetrazolium salts, it contains those of the general formula I

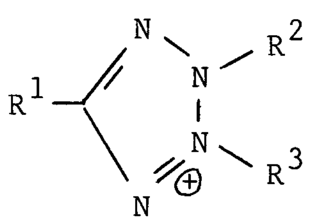 $A^{\ominus}$ (I)

in which $R^1$ signifies hydrogen, a carboxyl, alkyl, phenyl, nitrophenyl, dinitrophenyl, carboxyl-substituted phenyl or trialkylammonium phenyl radical, $R^2$ a phenyl, nitrophenyl, biphenylyl or naphthyl radical, $R^3$ a phenyl, carboxy-substituted phenyl, carboxy-substituted hydroxyphenyl or a dimethylthiazoyl radical and $A^{\ominus}$ a conventional counterion.

**7.** Reagent according to one of the preceding claims, characterised in that it additionally contains one or more electron transmitters.

**8.** Reagent according to one of claims 5 to 7, characterised in that it is present impregnated on an insoluble carrier.

**9.** Tetrazolium salts of the general formula I'

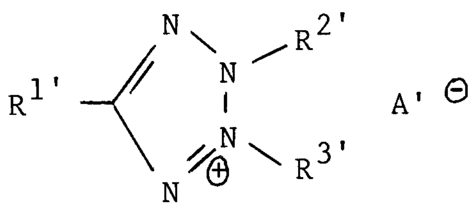

(I')

in which $R^{1'}$ signifies a carboxy group, an unsubstituted phenyl radical or one substituted in the 2- or 4-position by a carboxy group, disubstituted in the 2- and 4-positions by nitro groups or substituted in the 4-position by trimethylammonium, $R^{2'}$ an unsubstituted phenyl radical, $R^{3'}$ an unsubstituted phenyl radical or one substituted in the 2-position by a carboxyl group and $A'^{\ominus}$ a conventional counterion.

**10.** Process for the preparation of compounds of the general formula I'

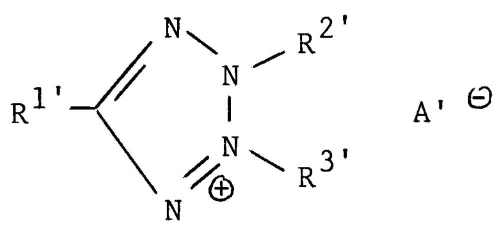

(I')

in which $R^{1'}$ signifies a carboxyl group, an unsubstituted phenyl radical or one substituted in the 2- or 4-position by a carboxy group, disubstituted in the 2-and 4-positions by nitro groups or substituted in the 4-position by trimethylammonium, $R^{2'}$ an unsubstituted phenyl radical, $R^{3'}$ an unsubstituted phenyl radical or one substituted in the 2-position by a carboxyl group and $A'^{\ominus}$ a conventional counterion, characterised in that either

a) aldehyde phenylhydrazones of the general formula II

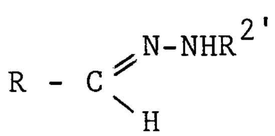

(II)

in which R signifies an esterified carboxyl group, an unsubstituted phenyl radical or a phenyl radical substituted in the 2- or 4-position by a carboxyl group, in the 2- and 4-positions by nitro groups or substituted in the 4-position by trimethylammonium and $R^{2'}$ an unsubstituted phenyl radical, are coupled under alkaline conditions with diazonium salts of the general formula III

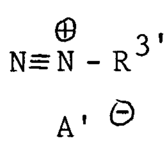

(III)

in which $R^{3'}$ signifies an unsubstituted phenyl radical or one substituted in the 2-position by a carboxyl group and $A'^{\ominus}$ a conventional counterion, to give formazanes of the general formula IV

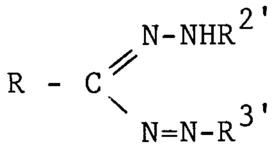

(IV)

in which R, $R^{2'}$ and $R^{3'}$ have the above-given meaning; or
b) aldehyde phenyl hydrazones of the general formula II'

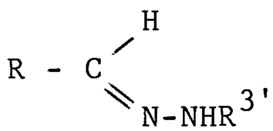

(II')

in which R signifies an esterified carboxyl radical, an unsubstituted phenyl radical or a phenyl radical substituted in the 2- or 4-position by a carboxyl group, disubstituted in the 2- and 4-positions by nitro groups or substituted in the 4-position by trimethylammonium and $R^{3'}$ an unsubstituted phenyl radical or one substituted in the 2-position of a carboxyl group, are coupled under alkaline conditions with diazonium salts of the general formula III'

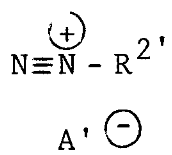

(III')

in which $R^{2'}$ signifies an unsubstituted phenyl radical and $A^{\ominus}$ a conventional counterion, to give formazanes of the general formula IV'

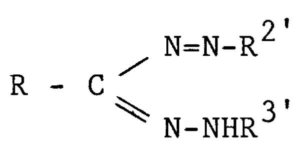

(IV')

in which R, $R^{2'}$ and $R^{3'}$ have the above-given meaning, and these are subsequently oxidised.

**Revendications**

1. Procédé pour la détermination de substrats ou d'activités enzymatiques, avec utilisation, comme réaction de mesure, d'une réaction redox détectable par photométrie, caractérisé en ce que l'on opère en présence d'un ou de plusieurs sels de tétrazolium supplémentaires, les sels de tétrazolium et leur formazane n'absorbant pas la lumière, ou seulement dans une proportion négligeable, à la longueur d'onde de la réaction de mesure.

2. Procédé selon la revendication 1, caractérisé en ce que, comme sel de tétrazolium supplémentaire, on utilise un composé de formule générale I

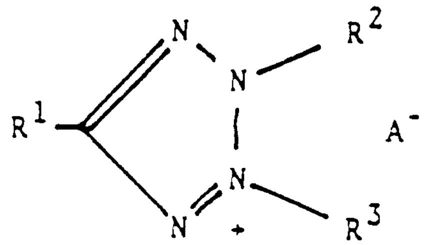

(I)

dans laquelle,

- $R^1$ représente l'hydrogène, un groupe carboxyle, alkyle, phényle, nitrophényle, dinitrophényle, phényle substitué par un groupe carboxyle, ou un groupe trialkylammoniumphényle,
- $R^2$ représente un groupe phényle, nitrophényle, biphénylyle ou naphtyle,
- $R^3$ représente un groupe phényle, un groupe phényle substitué par un groupe carboxyle, un groupe hydroxyphényle ou diméthylthiazolyle substitué par un groupe carboxyle, et
- $A^-$ représente un ion antagoniste usuel.

**3.** Procédé selon l'une des revendications 1 ou 2, caractérisé en ce que l'on utilise en outre un ou plusieurs agents de transfert d'électrons.

**4.** Procédé selon la revendication 3, caractérisé en ce que comme agent de transfert d'électrons, on utilise le méthosulfate de phénazine, l'éthosulfate de phénazine, la 8-diméthylamino-2,3-benzophénoxazine, le méthylsulfate de 1-méthoxy-5-méthylphénazinium ou le diaphorase.

**5.** Réactif pour la détermination enzymatique de substrats ou pour la détermination d'activités enzymatiques, contenant un système pour la détermination d'un substrat ou d'une enzyme à l'aide d'une réaction redox détectable par photométrie, comme réaction de mesure, caractérisé en ce qu'il contient en outre un ou plusieurs sels de tétrazolium supplémentaires lesquels, aussi bien que leur formazane correspondante formée lors d'une réduction, n' absorbent pas la lumière, ou seulement dans une proportion négligeable, à la longueur d'onde de la réaction de mesure.

**6.** Réactif selon la revendication 5, caractérisé en ce que comme sel de tétrazolium supplémentaire, on utilise les composés de formule générale I

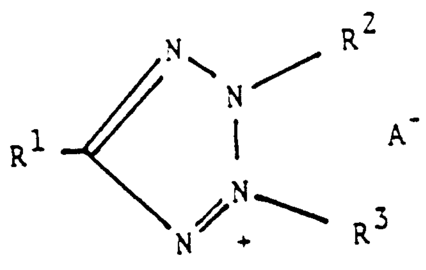

(I)

dans laquelle

- $R^1$ représente l'hydrogène, un groupe carboxyle, alkyle, phényle, nitrophényle, dinitrophényle, phényle substitué par un groupe carboxyle, ou un groupe trialkylammoniumphényle,
- $R^2$ représente un groupe phényle, nitrophényle, biphénylyle ou naphtyle,
- $R^3$ représente un groupe phényle, un groupe phényle substitué par un groupe carboxyle, un groupe hydroxyphényle substitué par un groupe carboxyle, ou un groupe diméthylthiazolyle et
- $A^-$ représente un ion antagoniste usuel.

**7.** Réactif selon l'une quelconque des revendications précédentes, caractérisé en ce qu'il contient en outre un ou plusieurs agents de transfert d'électrons.

**8.** Réactif selon l'une quelconque des revendications 5 à 7, caractérisé en ce qu'il se présente sous forme imprégnée sur un support insoluble.

**9.** Sel de tétrazolium de formule générale I'

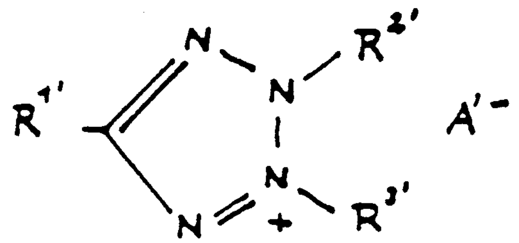

(I')

dans laquelle

- $R^{1'}$ représente un groupe carboxyle, un groupe phényle non substitué ou substitué en position 2 ou 4 par un groupe carboxyle, disubstitué en positions 2 et 4 par un groupe nitro ou substitué en position 4 par un groupe triméthylammonium,
- $R^{2'}$ représente un groupe phényle non substitué,
- $R^{3'}$ représente un groupe phényle non substitué ou substitué en position 2 par un groupe carboxyle et
- $A'^{-}$ représente un ion antagoniste usuel.

**10.** Procédé pour la préparation de composés de formule générale I'

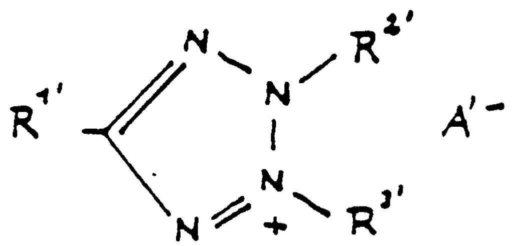

(I')

dans laquelle

- $R^{1'}$ représente un groupe carboxyle, un groupe phényle non substitué ou substitué en position 2 ou 4 par un groupe carboxyle, disubstitué en positions 2 et 4 par un groupe nitro ou substitué en position 4 par un groupe triméthylammonium,
- $R^{2'}$ représente un groupe phényle non substitué,
- $R^{3'}$ représente un groupe phényle non substitué ou substitué en position 2 par un groupe carboxyle et
- $A'^{-}$ représente un ion antagoniste usuel,

caractérisé en ce que

soit

a) on effectue la copulation d'aldéhydephénylhydrazones de formule générale II

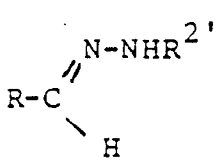

(II)

dans laquelle

R représente un groupe carboxyle estérifié, un groupe phényle non substitué ou un groupe phényle substitué en position 2 ou 4 par un groupe carboxyle, disubstitué en positions 2 et 4 par un groupe nitro ou substitué en position 4 par un groupe triméthylammonium et

$R^{2'}$ représente un groupe phényle non substitué,

avec des sels de diazonium de formule générale III

$$\overset{+}{N}\equiv N-R^{3'} \quad A'^{-} \qquad (III)$$

dans laquelle

$R^{3'}$ représente un groupe phényle non substitué ou substitué en position 2 par un groupe carboxyle et

$A'^{-}$ représente un ion antagoniste usuel,

en milieu alcalin pour donner des formazanes de formule générale IV

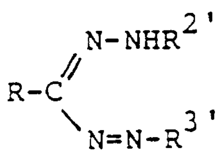

(IV)

dans laquelle R, $R^{2'}$ et $R^{3'}$ ont les significations mentionnées ci-dessus,

soit

b) on effectue la copulation d'aldéhydephénylhydrazones de formule générale II'

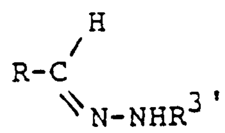

(II')

dans laquelle

R représente un groupe carboxyle estérifié, un groupe phényle non substitué ou un groupe phényle substitué en position 2 ou 4 par un groupe carboxyle, disubstitué en positions 2 et 4 par des groupes nitro ou substitué en position 4 par un groupe triméthylammonium et

$R^{3'}$ représente un groupe phényle non substitué ou substitué en position 2 par un groupe carboxyle,

avec des sels de diazonium de formule générale III'

$$\overset{+}{N}\equiv N-R^{2'} \quad A'^{-} \qquad (III')$$

dans laquelle

$R^{2'}$ représente un groupe phényle non substitué et

$A'^{-}$ représente un ion antagoniste usuel,

en milieu alcalin pour donner des formazanes de formule générale IV'

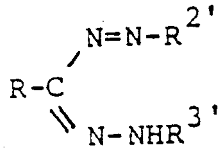

(IV')

dans laquelle
R, $R^{2'}$ et $R^{3'}$ ont les significations ci-dessus, et l'on oxyde ensuite les composés obtenus.

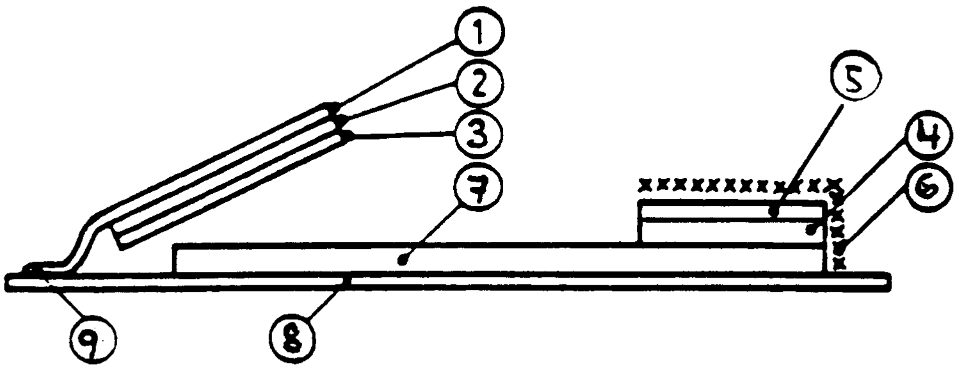
1
2
3
5
4
7
6
9
8